# FASCICULE DE BREVET EUROPEEN

(11) **EP 0 902 492 B1**
(45) Date de publication et mention de la délivrance du brevet: **14.11.2007**
(21) Numéro de dépôt: 98402242.6
(22) Date de dépôt: 10.09.1998
(51) Int. Cl.: H01M 6/18, H01M 10/40, H01M 6/16, C07C 381/10

(54) **Solvants et nouvelles compositions électrolytiques possédant un large domaine de stabilité et une conductivité élevée**
Lösungsmittel und neue Elektrolytzusammensetzungen mit breitem Stabilitätbereich und hoher Leitfähigkeit
Solvents and new electrolytic compositions having a large stability range and high conductivity

(30) Priorité: 11.09.1997 CA 2215849
(43) Date de publication de la demande: 17.03.1999
(60) Demande divisionnaire: 07014876.2
(73) Titulaire: CENTRE NATIONAL DE LA RECHERCHE SCIENTIFIQUE (CNRS), 75016 Paris (FR); UNIVERSITE DE MONTREAL, Montreal, Quebec H3T 1J4 (CA); ACEP INC., Montreal, Quebec H2Z 1A4 (CA)
(72) Inventeur: Michot, Christophe, 38000 Grenoble (FR); Brouillette, Dany, Montreal, Quebec H3K 1R7 (CA); Baril, Daniel, Montreal, Quebec H2G 2Y3 (CA); Bergeron, Jean-Yves, Longueuil, Quebec H4G 2H1 (CA); Armand, Michel, Montreal, Quebec H3T 1N2 (CA)
(74) Mandataire: L'Helgoualch, Jean

(56) Documents cités:
- EP-A- 0 125 093
- EP-A- 0 126 558
- EP-A- 0 339 284
- BE-A- 876 201
- DE-A- 2 300 084
- DE-A- 3 632 737
- US-A- 5 723 664
- CHEMICAL ABSTRACTS, vol. 72, no. 13, 1970 Columbus, Ohio, US; abstract no. 66338, S.P. VON HALASZ ET AL.: "prep of new aminosulfur monofluoride imides and aminosulfuroxide monofluoride imides" XP002089526 & CHEM. BER., vol. 103, no. 2, 1970, pages 594-602,

## Description

### DOMAINE DE L'INVENTION

Le domaine de l'invention est celui de nouveaux solvants polaires et de nouvelles compositions électrolytiques en découlant et possédant un domaine de stabilité élevé, tel que requis pour les applications dans le domaine électrochimique.

### ART ANTÉRIEUR

On connaît les solvants aprotiques polaires comme les carbonates cycliques ou linéaires, les éthers employés seuls ou en mélange, dans de nombreuses compositions électrolytiques. La stabilité de ces composés face à des potentiels très négatifs, proches de ceux des métaux alcalins, ou très positifs (≥ 4V par rapport à Li⁺/Li°), est loin d'être satisfaisante, et les batteries au lithium comprenant des électrolytes obtenus par dissolution d'un sel de lithium dans ces solvants posent de sérieux problèmes de sécurité. Les composés de types amide linéaire ou cyclique comme la diméthylformamide ou la N-méthylpyrrolidinone possèdent d'excellentes propriétés en tant que solvants mais s'oxydent à des potentiels encore bas, de l'ordre de 3,7 V par rapport à Li⁺ / Li°.

De nombreux matériaux d'électrodes positives, tels que les oxydes mixtes de métaux de transition et de lithium fonctionnent à des potentiels proches de 4 V par rapport à Li⁺/Li° et nécessitent donc des stabilités d'électrolytes nettement supérieures à cette valeur. Par exemple, les composés Li_{1-y}Co_{1-x-z}NiₓAl_{y}O₂ dans lesquels x + y ≤ 1 et z ≤ 0,3); les spinelles de manganèse Li_{1-α}Mn₂₋ₓO₄• Li_{1-α}Co_{1-x-y}NiₓAl_{y} dans lesquels 0 ≤ x + y ≤ 1 ; 0 ≤ y ≤ 0,3; 0 ≤ α ≤ 1 et M = Li, Mg, Al, Cr, Ni, Co, Cu, Ni, Fe.

Les brevets US 4,851,307 et US 5,063,124 décrivent une famille d'électrolytes mettant en oeuvre un sel, un polymère solvatant et un solvant aprotique de la famille des sulfamides de formule générale

R¹R²NSO₂R³R⁴

où R¹, R², R³ et R⁴, identiques ou différents, sont choisis indépendamment parmi les groupes C₁₋₁₀alkyles ou C₁₋₁₀oxaalkyles. Un exemple représentatif de ce groupe est la tétraéthylsulfamide (R¹ = R² = R³ = R⁴ = C₂H₅). Ces matériaux ont une stabilité accrue face à des agents réducteurs ou basiques présents ayant des potentiels proches de ceux des métaux alcalins. Ils sont par contre oxydables à des potentiels compris entre 3,8 et 4V par rapport à Li⁺ / Li°.

Les composés décrits dans le brevet européen EP 0 339 284 consistent en des composés diélectriques et isolants constitués par des perfluoro-acylamides ou perfluoro-sulfonamides R_{F}CONA¹A² et R_{F}SO₂NA¹A², où A¹ et A² sont des groupements alkyles. L'utilisation proposée de ces composés dans des condensateurs implique que ces matériaux n'ont pas de conductivité et que les impuretés et contaminations inéluctables, en particulier par des composés ioniques, n'induit pas d'augmentation de conductivité appréciable.

La publication de Sartori et al. dans un abrégé d'une rencontre de l'Electrochemical Society, Volume 97-1, mai 1997, décrit entre autres, que certains sulphonamides pourraient être utilises comme électrolyte dans une batterie ou dans un système de stockage d'énergie.

### SOMMAIRE DE L'INVENTION

La présente invention concerne une série de nouveaux solvants polaires et de nouvelles compositions électrolytiques en découlant et possédant un domaine de stabilité élevé, tel que requis pour les applications dans le domaine électrochimique. Plus spécifiquement, les solvants de la présente invention correspondent à la formule générale

R¹R²NSO₂F

dans laquelle
R¹ et R² sont identiques ou différents et représentent C₁₋₁₈alkyle, C₁₋₁₈ oxaalkyle, C₁₋₁₈ alkylène ou C₁₋₁₈oxaalkylène;

L'expression "essentiellement fluoré" signifie que le degré of fluoration de la chaîne est suffisant pour conférer des propriétés semblables à celles des chaînes entièrement perfluorées, telles que le caractère hydrophobe et des propriétés d'attracteur d'électrons. De préférence, au moins environ la moitié des atomes d'hydrogène de la chaîne sont remplacés par des atomes de fluor. L'expression "partiellement chlorés" signifie que parmi les composés partiellement fluorés, les atomes d'hydrogène restant sont au moins partiellement remplacés par des atomes de chlores.

### DESCRIPTION DÉTAILLÉE DE L'INVENTION

Dans la présente invention, des matériaux possédant une fonction amide très polaire sont utilisés comme base pour la préparation de compositions électrolytiques utiles pour les applications électrochimiques. Il a été trouvé que, de façon tout à fait inattendue, des groupements très fortement attracteurs d'électrons associés à la fonction amide permettent de maintenir un pouvoir solubilisant face à des composés ioniques, en particulier ceux dont la charge anionique est fortement délocalisée, et ainsi d'induire des conductivités ioniques élevées. En ajoutant un polymère polaire à ces compositions, on obtient des électrolytes ayant des propriétés mécaniques facilitant leur mis en oeuvre, en particulier sous forme de films, pour leur utilisation dans des dispositifs électrochimiques et augmentant leur sécurité de fonctionnement. Dépendant des quantités respectives de solvant polaire et de polymère dans les compositions électrolytiques, la consistance de celles-ci s'apparente à celle d'un gel ou à celle d'un polymère plastifié. En outre, les polymères peuvent être réticulés pour améliorer les propriétés mécaniques.

Par rapport aux matériaux de l'art antérieur, les nouvelles compositions électrolytiques de la présente invention possèdent une stabilité accrue, en particulier aux potentiels très anodiques, spécialement ceux excédant 4V par rapport à Li⁺ / Li°.

Les sels de faible énergie réticulaires préférentiels qui sont solubles dans les solvants polaires de la présente invention pour former des solutions conductrices incluent ceux ayant une charge délocalisée, tels que I⁻, ClO₄⁻, BF₄⁻, PF₆⁻, AsF₆⁻, SbF₆⁻, R_{F}SO₃⁻, XSO₂NSO₂X'⁻, (XSO₂)(X'SO₂)(Y)C⁻ et leurs mélanges, dans lesquels
- X et X' sont choisis parmi R_{F}, R_{F}CH₂O-, (R_{F})₂CHO-, (R_{F}CH₂)₂N-, R⁸, R⁹R¹⁰N-, avec la restriction qu'au moins un X ou X' est choisi parmi R_{F}, R_{F}CH₂O-, (R_{F})₂CHO-, (R_{F}CH₂)₂N-;
- Y = R_{F}, R_{F}SO₂ ou CN;
- R_{F} est tel que défini précédemment; et
- R⁸ à R¹⁰ sont identiques ou différents, et représentent C₁₋₁₈alkyles ou C₁₋₁₈ oxaalkyle; R_{F} et R⁸ -R¹⁰ pouvant faire partie d'une chaîne macromoléculaire. Sont aussi préférés les anions dérivés du 4,5-dicyano-1,2,3-triazole, du 3,5-bis(R_{F})-1,2,4-triazole, le tricyanométhane, le pentacyanocyclopentadiène et le pentakis(trifluorométhyl)cyclopentadiène et les anions dérivés de la cyanamide et du malononitrile, i.e., R_{F}SO₂NCN⁻, C(CN)₃⁻, R_{F}SO₂C(CN)₂⁻. Les cations préférentiels sont choisis parmi ceux dérivés des métaux alcalins, en particulier le lithium, des métaux alcalino-terreux, les cations organiques de type "onium", en particulier les ammonium, imidazolium, sulfonium, phosphonium et oxonium.

Parmi les compositions électrolytiques faisant l'objet de la présente invention, mentionnons celles contenant au moins un solvant polaire tel que défini précédemment en combinaison avec une ou plusieurs autres molécules polaires en tant que co-solvant. Parmi ces autres molécules polaires, citons les solvants susceptibles de former des mélanges compatibles, par exemple les éthers di-alkyliques de l'éthylène glycol, du diéthylène glycol, du triéthylène glycol, des polyéthylènes glycols ayant préférablement une masse compnse entre 400 et 2000; ou les esters, en particulier ceux de l'acide carbonique, linéaires ou cycliques, tels que le diméthylcarbonate, le méthyl-éthylcarbonate, le diéthylcarbonate, le carbonate d'éthylène, le carbonate de propylène; ou des esters comme la γ-butyrolactone, les nitriles comme le glutaronitrile, ou le 1,2,6-tricyanohexane Ces autres molécules polaires, ou co-solvant, peuvent être ajoutés seules au solvant de la présente invention, ou en mélange. Un exemple de mélange préférentiel est le mélange du carbonate d'éthylène avec un éther di-alkylique.

La présente invention inclut en outre les électrolytes solides obtenus par addition d'un polymère aux solvants ou mélange de solvants - co-solvants contenant au moins un sel en solution tels que définis ci-dessus. La proportion de polymère peut être choisie de manière à ce que le solvant agisse comme plastifiant du polymère, dans une fraction de 3 à 30% en poids, de préférence de 10 à 25% en poids. Les polymères préférés pour ces compositions sont ceux dont les unités monomères possèdent des unités solvatantes, tels ceux dérivés de l'oxyde d'éthylène, l'oxyde de propylène, l'épichlorohydrine, l'épifluorohydrine, le trifluoroépoxypropane etc. Le polymère peut aussi servir à constituer un gel quand la fraction massique du solvant et du sel est comprise entre 30 et 95% en poids, de préférence de 40 à 70%. Outre les polymères précités, ceux contenant des unités dérivées de l'acrylonitrile, du méthacrylate de méthyle, du fluorure de vinylidène, de la N-vinylpyrolidinone sont également avantageux, et peuvent être des homo- ou des co-polymères. En particulier, on peut citer les copolymères du fluorure de vinylidène et de l'hexafluoropropène. Un copolymère contenant de 5 à 30% molaire d'hexafluoropropène est particulièrement avantageux. Dans une variante, les polymères sont des polyélectrolytes incorporant dans la trame macromoléculaire des anions de type de ceux à charge delocalisée. Dans ces conditions, les charges négatives sont immobilisées et seules les contre-charges positives participent au processus de conduction ionique.

Les compositions électrolytiques de l'invention sont utilisables dans tous les cas où une grande stabilité est requise, plus particulièrement vis-à-vis de l'oxydation ou des potentiels très positifs. Un bon exemple est un générateur électrochimique dans lequel il est avantageux de disposer d'une force électromotrice élevée, et plus particulièrement les générateurs mettant en jeu l'ion lithium. Dans un tel système, l'électrode négative est constituée de lithium métallique, d'un de ses alliages, un composé d'insertion du carbone, en particulier du coke de pétrole ou du graphite, un oxyde à bas potentiel d'insertion tel que les spinelles de titane Li_{2x+1+3y}Tiₓ₊₅O₁₂ (x ≥ 0 et y ≤ 1), un nitrure double d'un métal de transition et de lithium comme Li₃₋ₓCoₓN, ou ayant la structure de type antifluorite comme Li₃FeN₂ ou Li₇MnN₄.

Les matériaux d'électrode positive sont choisis parmi les composés d'insertion, les polydisulfures ou les oxocarbones. Parmi les composés d'insertion, sont préférés l'oxyde de vanadium, et de façon préférentielle celui de formule VOₓ où 2 ≤ x ≤ 2,5, l'oxyde mixte de lithium et de vanadium LiV₃O₃; les oxydes doubles de cobalt et de lithium partiellement substitués de formule générale Li₁₋ₐ Co_{1-x-y}NₓAl_{y} dans lequel 0 ≤ x + y ≤1 ; 0 ≤ y ≤ 0,3 ; 0 ≤ α ≤ 1; les spinelles de manganèse partiellement substitués de formule générale Li_{1-α}Mn_{2-z}M_{z}O₄ dans lequel 0≤ z≤ 1 et M = Li, Mg, Al, Cr, Ni, Co, Cu, Ni, Fe; et les phosphates doubles de structure olivine ou Nasicon tels que Li₁₋ₐFe₁₋ₓMnₓPO₄, Li₁₋ₐ₊₂ₓFe₂P₁₋ₓSₓO₄, dans lesquels x ≥ 0 et α ≤ 1. Les matériaux d'électrode de type oxocarbones sont de préférence choisis parmi les sels de l'acide rhodizonique, les polydisulfure sont choisis parmi ceux dérivés de l'oxydation du dimercaptoéthane, du 2,5-dimercapto-1,3,4-thiadiazole, du 2,5-dimercapto-1,3,4-oxaadiazole, du 1,2-dimercaptocyclobutène-3,4-dione.

Les générateurs électrochimiques utilisant les compositions électrolytiques selon l'invention utilisent de préférence des électrolytes solides, de type plastifié ou sous forme de gel. Dans une réalisation préférée de l'invention, au moins une des électrodes est sous Forme de composite contenant le ou les matériaux d'électrodes mélangés à la composition électrolytique et du carbone sous forme divisée, tel le noir de Shawinigan^{®}, le Ketjenblack^{®}, le graphite.

Une autre application de l'invention est celle des supercapacités, dans lesquelles au moins une électrode est constituée de carbone de haute surface spécifique et l'énergie électrique est stockée par la capacité de la double couche entre le matériau carboné et l'électrolyte. Dans un mode de réalisation préférentiel, les deux électrodes sont symétriquement faites à base de carbone de haute surface spécifique, et ce matériau est mis en oeuvre sous forme de composite en mélange avec l'électrolyte. Une autre possibilité consiste à utiliser un matériau d'électrode contenant au moins un polymère possédant des doubles liaisons conjuguées. Dans un mode préféré de réalisation, le polymère conjugué peut présenter trois degrés d'oxydation, obtenus par réduction (dit dopage "n") concomitant à une injection d'électrons et de cations, ou par oxydation (dit dopage "p") concomitant à une extraction d'électrons et injection d'anions, à partit de la forme neutre. Les polymères à base de phényl-3-thiophène, en particulier le poly(4-fluorophényl-3-thiophène) sont particulièrement préférés.

Les exemples fournis ci-dessous le sont afin d'illustrer des modes de réalisations préférentiels de la présente invention, et ne devraient en aucun cas être considérés comme limitant la portée de l'invention.

### Exemple Comparatif 1

Du trifluoroéthanol (18.2 mL, 25 mml) est dissous dans 100 mL d'éther et cette solution est ajoutée à 7 g d'hydrure de sodium. Lorsque la génération de gaz a cessé, la solution est centrifugée, et le liquide surnageant clair est ajouté à 0°C à 35 µg (25 mml) de chlorure de diméthylsulfamoyle dissous dans 100 mL d'éther sec sous agitation. Un précipité blanc de chlorure de sodium est alors formé et la réaction est complète après 2 heures. La suspension résultante est filtrée et l'éther est évaporé à l'aide d'un évaporateur rotatif. Le résidu est placé dans 50 ml de dichlorométhane et lavé avec une solution aqueuse 10% d'acide chlorhydrique. La phase organique est ensuite séparée et séchée avec du sulphate de magnésium anhydre. Le produit résultant, le N,N diméthylsulfamate de trifluoéthyle est distillé sous pression réduite. RMN: ¹⁹F: triplet δ = 74.7 ppm, *J_{HF}* = 8.1 Hz; ¹H: quadruplet δ = 4.66 (2H), singulet δ = 3.6 (6H). La conductivité des sels de lithium de la bis(trifluorométhanesulfonimide) (CF₃SO₂)₂NLi en solution dans ce solvant est donnée en fonction de la concentration dans le Tableau 1.

**Tableau 1**

| molalité (mol.kg⁻¹) | conductivité κₛₚ (S.cm⁻¹) |
|---|---|
| 0.265 | 0.634 |
| 0.506 | 0.922 |
| 0.898 | 1.025 |
| 1.160 | 0.796 |

Le domaine de stabilité électrochimique a été mesuré par voltammétrie cyclique sur microélectrode de platine de 15 µm de diamètre pour l'exploration des potentiels anodiques, de nickel pour les potentiels cathodiques. Le domaine de stabilité est de 0 à 5.2 V vs. Li⁺ / Li°. La variation de la conductivité en fonction de la température est donnée dans le Tableau 2 suivant pour une concentration de 0.898 mol.kg⁻¹.

**Tableau 2**

| T (°C) | κₛₚ(S.cm⁻¹) | T (°C) | κₛₚ(S.cm⁻¹) |
|---|---|---|---|
| 14.90 | 0.753 | 30.29 | 1.203 |
| 14.92 | 0.7550 | 35.39 | 1.415 |
| 19.93 | 0.882 | 40.81 | 1.657 |
| 25.11 | 1.030 | | |

### Exemple 1

107.4 mL de de chlorure de diméthylsulfamoyle sont mis au reflux sous azote en présence de 70 g de fluorure de potassium et 10 ml d'eau. Le mélange est refoidi et extrait par le dichlorométhane, séché avec du sulfate de magnésium et distillé. Le composé (CH₃)₂NSO₁F obtenu a une constante diélectrique supérieure à 30. Le domaine de stabilité tel que déterminé par voltammétrie cyclique est de 5 V vs. Li+/Li°. Le sel de lithium de la fluorosulfonimide (FSO₂)₁NLi est soluble dans ce milieu et sa conductivité à 25°C est supérieure à 1 mScm⁻¹ dans la plage de concentration 0.5 à 1 mole.kg⁻¹.

### Exemple Comparatif 2

À 6.3 mL de 1,1,1,-3,3,3,-hexafluoropropanol dans 25 mL l'éther anhydre sont ajoutés 1.6 g d'hydrure de sodium. Lorsque le dégagement d'hydrogène a cessé, la solution est centrifugée, et au liquide surnageant sont ajoutés 8.6 µg (60 mml) de chlorure de diméthylsulfamoyle dissous dans 25 mL d'éther sec à 0°C sous agitation. Un précipité blanc de chlorure de sodium est alors formé et la réaction est complète après 2 heures. La suspension résultante est filtrée et l'éther est évaporé à l'aide d'un évaporateur rotatif. Le résidu est placé dans 20 mL de dichlorométhane et lavé avec une solution aqueuse 10% d'acide chlorhydrique. La phase organique est ensuite séparée et séchée avec du sulphate de magnésium anhydre. Le produit résultant, le N,N-diméthylsulfamate d'hexafluoropropyle, est obtenu par évaporation du dichlorométhane et purifié par distillation sous pression réduite. Sa constante diélectrique est supérieure à 20, et les solutions de sels de bis(triflucrométhanesalfonimide) (NC₂H₅)₄(CF₃SO₂)₂N en solution dans ce solvant est comprise entre 5 x 10⁻⁴ et 2 x 10⁻³ Scm⁻¹ à 25°C dans la gamme de concentration 0,2 à 1 mole.kg⁻¹.

### Exemple Comparatif 3

À 15.76 g de chlorure d'éthylméthylsulfamoyle dissous dans 100 mL de tétrahydrofuranne sont ajoutés 4.2 g de cyanamide et 11.22 g de diazabicyclo 2,2,2-octane (DABCO). Après avoir maintenu l'agitation pendant 8 heures à température ordinaire, sont ajoutés goutte à goutte 13 g de chlorure d'oxalyle dans 40 mL de tétrahydrofuranne anhydre. Après la fin du dégagement gazeux (CO + CO₂), le chlorhydrate de DABCO est séparé par filtration et le filtrat est évaporé sous pression réduite et repris dans 50 mL d'acétonitrile auxquels sont ajoutés à 0°C 12.2 g d'éthylméthylamine. Le précipité de chlorure d'éthyl-méthylammonium est séparé et le solvant est évaporé sous vide. Le composé polaire : 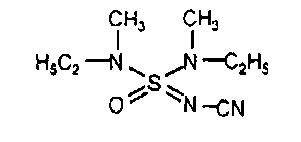 est mis en solution dans le dichlorométhane et lavé à l'eau contenant 2% d'acide chlorhydrique, puis 5% de bicarbonate de sodium. Après élimination du dichlorométhane, le produit est distillé sous pression réduite. Ce produit est un solvant des sels d'anions délocalisés, en particulier les imides perfluorées.

### Exemple Comparatif 4

33 g de 1,1-dimcthylsulfamide (CH₃)₂SO₂NH₂ et 6 g de soude dans 200 ml d'eau sont portés à ébullition pendant 2 heures. Le produit de la réaction, le sel de sodium de la bis(diméthylaminosulfonimide), i.e., Na[N(SO₂N(CH₃)₂)] est obtenu par évaporation de l'eau et purifié par recristallisation dans l'éthanol. 25 g de ce sel en suspension dans 100 mL d'acétonitrile anhydre sont traités par 9 mL de chlorure d'oxalyle. Après la fin de la réaction, i.e., du dégagement gazeux, la suspension est refroidie à 0°C et sont ajoutés 20.7 mL de diéthylamine dans 50 mL d'acétonitrile. Après avoir maintenu l'agitation pendant 4 heures à température ordinaire, le mélange est filtré et l'acétonitrile est évaporé sous pression réduite. Le liquide résultant est mis ne solution dans le dichlorométhane et lavé à l'eau contenant à 2% d'acide chlorhydrique, puis 5% de bicarbonate de sodium. La solution est passée sur une colonne d'alumine active et le dichlorométhane est évaporé sous vide. Le solvant polaire: 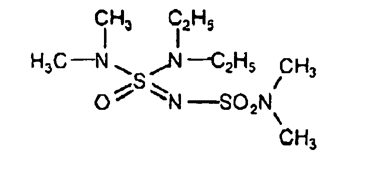 est maintenu anhydre par addition d'hydrure de lithium.

### Exemple Comparatif 5

Un générateur électrochimique constitué d'une électrode négative de lithium de 25 µm sur support de nickel de 10 µm, d'une électrode positive composite contenant 78 % en poids d'oxyde de vanadium V₂O₅, 8% de noir de carbone (Ketjenblack^{®}) et 14% de copolymère de fluorure de vinylidène et d'hexafluoropropène sur un collecteur de nickel (10 µm) a été réalisé. La capacité de l'électrode positive ainsi obtenue par épandage à partir d'une suspension dans du cyclohexanone est de 2.8 mAh/cm². L'électrolyte est constitué par une solution de 0.15 M.kg⁻¹ de Li(CF₃SO₂)₂N dans le composé polaire de l'exemple 1 dans un séparateur poreux en polypropylène de type Celgard^{®}. Le générateur a été cyclé sur 150 cycles entre 1.6 et 3.4V à C/3.7 en maintenant un rapport des capacités de charge et de décharge égal à 1 et un taux d'utilisation > 75% sur 30 cycles. La chute ohmique est restée comprise entre 20 et 120 mV.

### Exemple Comparatif 6

Un générateur électrochimiques de type "rocking chair" a été construit avec deux électrodes composites similaires à celles de l'Exemple 6. Le matériau de l'électrode négative est le spinelle de lithium et de titane Li₄Ti₅O₁₂ pour une capacité surfacique de 2.6 mAh.cm⁻². Le matériau de l'électrode positive est le cobaltite de lithium pour une capacité surfacique de 2.4 mAh.cm⁻². L'électrolyte est constitué d'une manière similaire à celui de l'Exemple 6 par une solution de 0,15 M.kg⁻¹ de Li(CF₃SO₂)₂N dans le composé polaire de l'Exemple 1 dans un séparateur poreux en polypropylène de type Celgard^{®}. Le générateur a été cyclé sur 500 cycles entre 1,5 et 3,3 V à C/4 en maintenant un rapport des capacités de charge et de décharge égal à 1 et un taux d'utilisation de 80%.

### Exemple Comparatif 7

Un générateur électrochimiques de type supercapacité a été construit avec deux électrodes composites symétriques de carbone de haute surface spécifique (680 m².g⁻¹) et de fibres de nickel sur un support de nickel et liées par un copolymère de fluorure de vinylidène et d'hexatluoropropène. L'électrolyte est constitué par un gel à 75% en poids d'une solution molaire de fluorosulfonimidure de tétraéthylammonium (C₂H₅)₄N[(CF₃SO₂)₂N] dans le même polymère. La capacité du système ainsi construit est de 1.2 F.g⁻¹ sur 12000 cycles effectuées entre 0 et 2.5 V.

### Exemple Comparatif 8

Un électrolyte polymère a été préparé par plastification d'un copolymère d'oxyde d'éthylène et d'allylglycidyl-éther contenant le sel de lithium du diméthylaminosulfonyl-trifluorométhanesulfonimide Li[(CH₃)₂SO₂NSO₂CF₃] dans un rapport oxygène des fonctions éther du polymère / Li de 14 :1 par le composé polaire de l'Exemple 5 dans un rapport de poids de 65:35. Cet électrolyte possède une conductivité de 10⁻⁴ Scm⁻¹à 25°C et un domaine de stabilité électrochimique de 0 à 4V vs. Li⁺/Li°. Le plastifiant n'a pas de pression de vapeur appréciable en dessous de 120°C. Cet électrolyte peut être réticulé par une source de radicaux libre pour donner des élastomères de bonne tenue mécanique.

Bien que la présente invention ait été décrite à l'aide de mises en oeuvre spécifiques, il est entendu que plusieurs variations et modifications peuvent se greffer aux dites mises en oeuvre, et la présente demande vise à couvrir de telles modifications usages ou adaptations de la présente invention suivant, en général, les principes de l'invention et incluant toute variation de la présente description qui deviendra connue ou conventionnelle dans le champ d'activité dans lequel se retrouve la présente invention, et qui peut s'appliquer aux éléments essentiels mentionnés ci-haut, en accord avec la portée des suivantes.

## Revendications

1. Composé aprotique polaire ayant des propriétés de solvant et répondant à la formule R¹R²NSO₂F
dans laquelle :
- R¹ et R² sont identiques ou différents et représente un groupe alkyle, un groupe oxaalkyle, un groupe alkylène ou un groupe oxaalkylène, lesdits groupes ayant de 1 à 18 atomes de carbone.

2. Composition électrolytique comprenant un composé polaire selon la revendication 1, et un sel soluble dans ledit composé polaire.

3. Composition électrolytique selon la revendication 2, comprenant en outre au moins un polymère.

4. Composition électrolytique selon la revendication 2 ou 3, **caractérisée en ce que** l'anion du sel soluble est choisi parmi I⁻, ClO₄⁻, BF₄⁻, PF₆⁻, AsF₆⁻, SbF₆⁻, R_{F}SO₃⁻, R_{F}SO₂NCN⁻, C(CN)₃⁻, R_{F}SO₂(CN)₂⁻, XSO₂NSO₂X'⁻,(XSO₂)(X'SO₂)(Y)C⁻, les anions dérivés du 4,5-dicyano-1,2,3-triazole, du 3,5-bis(R_{F})-1,2,4-triazole, du tricyanométhane, du pentacyanocyclopentadiène et du pentakis(trifluorométhyl)cyclopentadiène, et leurs mélanges, dans lesquels :
- X et X' sont choisis parmi R_{F}, R_{F}CH₂O-, (R_{F})₂CHO-, (R_{F}CH₂)₂N-R⁸, R⁹R¹⁰N-, avec la restriction qu'au moins un X ou X' est R_{F}, R_{F}CH₂O-, (R_{F})₂CHO-, (R_{F}CH₂)₂N- ;
- Y est R_{F}, R_{F}SO₂ ou CN ;
- R_{F} est un atome de fluor, un groupe alkyle, un groupe oxaalkyle ou un groupe azaalkyle, lesdits groupes ayant de 1 à 4 atomes de carbone, et peut faire partie d'une chaîne macromoléculaire ;
- R⁸ à R¹⁰ sont identiques ou différents, et représentent un groupe alkyle ou un groupe oxaalkyle, lesdits groupes ayant de 1 à 18 atomes de carbone, R⁸ à R¹⁰ pouvant faire partie d'une chaîne macromoléculaire.

5. Composition électrolytique selon l'une des revendications 1 à 4, **caractérisée en ce que** le cation du sel soluble est choisi parmi les cations dérivés des métaux alcalins, des métaux alcalino-terreux, les cations organiques de type "onium", en particulier les ammonium, imidazolium, sulfonium, phosphonium, oxonium et leurs mélanges.

6. Composition électrolytique selon la revendication 5, **caractérisée en ce que** le cation est au moins en partie du lithium.

7. Composition électrolytique selon l'une des revendications 2 à 6, **caractérisée en ce qu'**elle comprend, en plus du composé polaire, un co-solvant.

8. Composition électrolytique selon la revendication 7, **caractérisée en ce que** le co-solvant est aprotique et polaire, et choisi parmi les éthers di-alkyliques de l'éthylène glycol, du diéthylène glycol, du triéthylène glycol, des polyéthylène glycols; les esters, en particulier ceux de l'acide carbonique, linéaires ou cycliques tels le diméthylcarbonate, le méthyl-éthylcarbonate, le diéthylcarbonate, le carbonate d'éthylène, le carbonate de propylène; les esters comme la γ-butyrolactone, les nitriles comme le glutaronitrile, le 1,2,6-tricyanohexane ainsi que les mélanges des composés précités, et les amides comme le diméthyl formamide, la N-méthylpyrrolidinone.

9. Composition électrolytique selon la revendication 8, **caractérisée en ce que** la masse du polyéthylène glycol est comprise entre 400 et 2000.

10. Composition électrolytique selon la revendication 3, **caractérisée en ce que** le polymère est choisi parmi ceux dont les unités monomères sont dérivées de l'oxyde d'éthylène, de l'oxyde de propylène, de l'épichlorhydrine, de l'épifluorhydrine, du trifluoroépoxypropane,de l'acrylo-nitrile, du méthacrylate de méthyle, du fluorure de vinylidène, de la N-vinylpyrolidinone,de l'hexafluoro-propène, chacun sous forme d'homo- ou de co-polymère, et leurs mélanges.

11. Composition électrolytique selon la revendication 8, **caractérisée en ce qu'**elle est plastifiée ou sous forme de gel.

12. Composition électrolytique selon la revendication 10, **caractérisée en ce qu'**au moins un des polymère est un polyélectrolyte incorporant dans la trame macromoléculaire des anions de type de ceux à charge délocalisée.

13. Générateur électrochimique **caractérisé en ce qu'**il utilise comme électrolyte une composition électrolytique selon la revendication 3.

14. Générateur électrochimique selon la revendication 13, **caractérisé en ce que** l'électrode négative contient du lithium métallique, un de ses alliages, un composé d'insertion du carbone, un oxyde à bas potentiel d'insertion, un nitrure double d'un métal de transition et de lithium, ou leurs mélanges.

15. Générateur électrochimique selon la revendication 14 dans lequel le composé d'insertion du carbone est du coke de pétrole ou de graphite, et dans lequel l'oxyde à bas potentiel d'insertion est un spinelle de titane

16. Générateur électrochimique selon la revendication 14, en ce que l'électrode positive contient de l'oxyde de vanadium, de l'oxyde mixte de lithium et de vanadium, un oxyde double de cobalt et de lithium, un spinelle de manganèse; un phosphate double de structure olivine ou Nasicon, un sel de l'acide rhodizonique, un polydisulfure dérivé de l'oxydation du dimercaptoéthane, du 2,5-dimercapto-1,3,4-thiadiazole, du 2,5-dimercapto-1,3,4-oxadiazole, du 1,2-dimercaptocyclobutène-3,4-dione ou leurs mélanges.

17. Système de stockage de l'énergie électrique de type supercapacité **caractérisé en ce qu'**il utilise comme électrolyte une composition électrolytique selon la revendication 3.

## Claims

1. An aprotic polar compound having solvent properties, and of the general formula R¹R²NSO₂F wherein R¹ and R² are the same or different and are an alkyl group, an oxaalkyl group, an alkylene group or an oxaalkylene group, said groups having 1 to 18 carbon atoms.

2. An electrolytic composition comprising a polar compound according to claim 1, and a salt soluble in said compound.

3. An electrolytic composition according to claim 2, further comprising at least one polymer.

4. An electrolytic composition according to claims 2 or 3, **characterized in that** the anion of the soluble salt is selected from I⁻, ClO₄⁻, BF₄⁻, PF₆⁻, AsF₆⁻, SbF₆⁻, R_{F}SO₃⁻, R_{F}SO₂NCN⁻, C(CN)₃⁻, R_{F}SO₂(CN)₂⁻, XSO₂NSO₂X'⁻,(XSO₂)(X'SO₂)(Y)C⁻, anionic derivatives of 4,5-dicyano-1,2,3-triazole, 3,5-bis(R_{F})-1,2,4-triazole, tricyanomethane, pentacyanocyclopentadiene or of pentakis(trifluoromethyl)cyclopentadiene, and mixtures thereof, wherein :
- X and X' are selected from R_{F}, R_{F}CH₂O-, (R_{F})₂CHO-, (R_{F}CH₂)₂N-, R⁸, R⁹R¹⁰N-, with the provision that at least one of X or X' is R_{F}, R_{F}CH₂O-, (R_{F})₂CHO-, (R_{F}CH₂)₂N- ;
- Y is R_{F}, R_{F}SO₂ or CN ;
- R_{F} is a fluor atom, an alkyl group, an oxaalkyl group or an azaalkyl group, said groups having 1 to 4 carbon atoms, and can be part of a macromolecular chain ;
- R⁸ to R¹⁰ are the same or different, and are an alkyl group, or an oxaalkyl group, said groups having 1 to 18 carbon atoms, and R⁸ to R¹⁰ can be part of a macromolecular chain.

5. An electrolytic composition according to any of claims 1 to 4, **characterized in that** the cation of the soluble salt is selected from cations derived from alkali metals, alkaline earth metals, organic onium cations, particularly ammonium, imidazolium, sulfonium, phosphonium, oxonium, and mixtures thereof.

6. An electrolytic composition according to claim 5, **characterized in that** the cation is at least partly lithium.

7. An electrolytic composition according to any of claims 1 to 6, **characterized in that** it further comprises a co-solvent, in addition to the polar compound.

8. An electrolytic composition according to claim 7, **characterized in that** the co-solvent is aprotic and polar, and comprises diallkyl ethers of ethylene glycol, diethylene glycol, triethylene glycol, polyethylene glycols ; esters, particularly those of straight chain or cyclic carbonic acids, such as dimethylcarbonate, methyl-ethyl carbonate, diethyl carbonate, ethylene carbonate, propylene carbonate ; esters such as γ-butyrolactone, nitriles such as glutaronitrile, 1,2,6-tricyanohexane and mixtures thereof, and amides such as dimethyl formamide, N-methylpyrrolidinone.

9. An electrolytic composition according to claim 8, **characterized in that** the weight of polyethylene glycol is between 400 and 2000.

10. An electrolytic composition according to clam 3, **characterized in that** the polymer is selected from those comprising monomer units derives from ethylene oxide, propylene oxide, epichlorhydrine, epifluorhydrine, trifluoroepoxypropane, acrylo-nitrile, methylmethacrylate, vinylidene fluoride, N-vinylpyrolidinone, hexafluoro-propene, either as homo- or co-polymers, and mixtures thereof.

11. An electrolytic composition according to claim 8, **characterized in that** it is plasticized or in the form of a gel.

12. An electrolytic composition according to claim 10, **characterized in that** at least one polymer is a polyelectrolyte comprising a macromolecular chain and having anions wherein the charge is delocalized.

13. An electrochemical generator **characterized in that** is uses as the electrolyte, an electrolytic composition according to claim 3.

14. An electrochemical generator according to claim 13, **characterized in that** the negative electrode comprises metallic lithium, one of its alloys, a carbon derivative, an oxide with low potential of intercalation, a double nitride of transition metal of lithium, or mixtures thereof.

15. An electrochemical generator according to claim 14 wherein the carbon derivative is petroleum coke or graphite, et the oxide with low potential of intercalation is a titanium spinell.

16. An electrochemical generator according to claim 14, **characterized in that** the positive electrode comprises vanadium oxide, mixed oxide of lithium and vanadium, double oxide of cobalt and lithium, a manganese spinel; a double phosphate having olivine or Nasicon structure, a rhodizonic acid salt, a polydisulfide derived from oxidation of dimercaptoethane, 2,5-dimercapto-1,3,4-thiadiazole, 2,5-dimercapto-1,3,4-oxadiazole, 1,2-dimercaptocyclobutene-3,4-dione or mixtures thereof..

17. Supercapacitor as an electrical energy storage system, **characterized in** using an electrochemical composition according to claim 3.

## Patentansprüche

1. Aprotische polare Verbindung, die Lösungsmitteleigenschaften aufweist und der Formel R¹R²NSO₂F entspricht, worin R¹ und R² gleich oder verschieden sind und für eine Alkylgruppe, eine Oxyalkylgruppe, eine Alkylengruppe oder eine Oxyalkylengruppe stehen, wobei diese Gruppen 1 bis 18 Kohlenstoffatome aufweisen.

2. Elektrolytzusammensetzung, umfassend eine polare Verbindung nach Anspruch 1 sowie ein in diesem polaren Gemisch lösliches Salz.

3. Elektrolytzusammensetzung nach Anspruch 2, die unter anderem zumindest ein Polymer umfasst.

4. Elektrolytzusammensetzung nach Anspruch 2 oder 3, **dadurch gekennzeichnet, dass** das Anion des löslichen Salzes aus Folgenden ausgewählt ist: I⁻, ClO₄⁻, BF₄⁻, PF₆⁻, AsF₆⁻, SbF₆⁻, R_{F}SO₃⁻, R_{F}SO₂NCN⁻, C(CN)₃⁻, R_{F}SO₂(CN)₂⁻, XSO₂NSO₂X'⁻, (XSO₂)(X'SO₂)(Y)C⁻, den von 4,5-Dicyano-1,2,3-triazol, 3,5-Bis(R_{F})-1,2,4-triazol, Tricyanomethan, Pentacyanocyclopentadien und Pentakis(trifluormethyl)cyclopentadien abgeleiteten Anionen und Gemischen daraus, worin:
- X und X' aus R_{F}, R_{F}CH₂O-, (R_{F})₂CHO-, (R_{F}CH₂)₂N-, R⁸, R⁹R¹⁰N- ausgewählt sind, mit der Einschränkung, das zumindest X oder X' R_{F}, R_{F}CH₂O-, (R_{F})₂CHO- oder (R_{F}CH₂)₂N- ist;
- Y R_{F}, R_{F}SO₂ oder CN ist;
- R_{F} ein Fluoratom, eine Alkylgruppe, eine Oxyalkylgruppe oder eine Azaalkylgruppe ist, wobei diese Gruppen 1 bis 4 Kohlenstoffatome aufweisen, und Teil einer makromolekularen Kette sein kann;
- R⁸ bis R¹⁰ gleich oder verschieden sind und für eine Alkylgruppe oder eine Oxyalkylgruppe stehen, wobei diese Gruppen 1 bis 18 Kohlenstoffatome aufweisen und R⁸ bis R¹⁰ Teil einer makromolekularen Kette sein können.

5. Elektrolytzusammensetzung nach einem der Ansprüche 1 bis 4, **dadurch gekennzeichnet, dass** das Kation des löslichen Salzes aus den von Alkalimetallen, Erdalkalimetallen stammenden Kationen, den organischen Kationen des "Onium"-Typs, insbesondere aus Ammonium, Imidazolium, Sulfonium, Phosphonium, Oxonium, und Gemischen daraus ausgewählt ist.

6. Elektrolytzusammensetzung nach Anspruch 5, **dadurch gekennzeichnet, dass** das Kation zumindest teilweise von Lithium stammt.

7. Elektrolytzusammensetzung nach einem der Ansprüche 2 bis 6, **dadurch gekennzeichnet, dass** diese zusätzlich zu der polaren Verbindung ein Co-Solvens umfasst.

8. Elektrolytzusammensetzung nach Anspruch 7, **dadurch gekennzeichnet, dass** das Co-Solvens aprotisch und polar ist und aus Ethylenglykoldialkylethern, Diethylenglykoldialkylethern, Triethylenglykoldialkylethern, Polyethylenglykoldialkylethern; unverzweigten oder zyklischen Estern, insbesondere Carbonsäureestern, wie z.B. Dimethylcarbonat, Methylethylcarbonat, Diethylcarbonat, Ethylencarbonat, Propylencarbonat; Estern, wie z.B. γ-Butyrolacton; Nitrilen, wie z.B. Glutaronitril, 1,2,6-Tricyanohexan, sowie Gemischen der zuvor genannten Verbindungen und Amiden, wie z.B. Dimethylformamid, N-Methylpyrrolidinon, ausgewählt ist.

9. Elektrolytzusammensetzung nach Anspruch 8, **dadurch gekennzeichnet, dass** die Masse des Polyethylenglykols zwischen 400 und 2000 liegt.

10. Elektrolytzusammensetzung nach Anspruch 3, **dadurch gekennzeichnet, dass** das Polymer aus jenen ausgewählt ist, deren Monomereinheiten von Ethylenoxid, Propylenoxid, Epichlorhydrin, Epifluorhydrin, Trifluorepoxypropan, Acrylnitril, Methylmethacrylat, Vinylidenfluorid, N-Vinylpyrrolidinon, Hexafluorpropen, jeweils in Form eines Homo- oder Copolymers, und deren Gemischen stammen.

11. Elektrolytzusammensetzung nach Anspruch 8, **dadurch gekennzeichnet, dass** sie plastisch oder in Gelform vorliegt.

12. Elektrolytzusammensetzung nach Anspruch 10, **dadurch gekennzeichnet, dass** zumindest eines der Polymere ein Polyelektrolyt ist, der im makromolekularen Gerüst Anionen des Typs mit delokalisierter Ladung umfasst.

13. Elektrochemischer Generator, in dem eine Elektrolytzusammensetzung nach Anspruch 3 als Elektrolyt eingesetzt wird.

14. Elektrochemischer Generator nach Anspruch 13, **dadurch gekennzeichnet, dass** die negative Elektrode metallisches Lithium, eine seiner Legierungen, eine Verbindung mit eingelagertem Kohlenstoff, ein Oxid mit geringer Einlagerungsfähigkeit, ein Übergangsmetall-Lithium-Doppelnitrid oder Gemische daraus umfasst.

15. Elektrochemischer Generator nach Anspruch 14, worin die Kohlenstoffeinlagerungsverbindung Rohöl- oder Graphitkoks ist und das Oxid mit geringer Einlagerungsfähigkeit ein Titanspinell ist.

16. Elektrochemischer Generator nach Anspruch 14, worin die positive Elektrode Vanadiumoxid, Lithium-Vanadium-Mischoxid, ein Cobalt-Lithium-Doppeloxid, ein Manganspinell, ein Doppelphosphat einer Olivinstruktur oder Nasicon, ein Rhodizonsäuresalz, ein Polydisulfidderivat der Oxidation von Dimercaptoethan, 2,5-Dimercapto-1,3,4-thiadiazol, 2,5-Dimercapto-1,3,4-oxadiazol, 1,2-Dimercaptocyclobuten-3,4-dion oder Gemische davon umfasst.

17. Speichersystem des Superkapazitätstyps für elektrische Energie, **dadurch gekennzeichnet, dass** es als Elektrolyt eine Elektrolytzusammensetzung nach Anspruch 3 einsetzt.
